# EUROPÄISCHE PATENTSCHRIFT

(11) **EP 1 274 671 B1**
(45) Veröffentlichungstag und Bekanntmachung des Hinweises auf die Patenterteilung: **07.06.2006**
(21) Anmeldenummer: 01915332.9
(22) Anmeldetag: 14.03.2001
(51) Int. Cl.: C07C 45/50, C07C 45/79, C22B 3/00

(54) **VERFAHREN ZUR WIEDERGEWINNUNG VON RHODIUM AUS DEN PRODUKTEN DER HYDROFORMYLIERUNG**
METHOD FOR RECOVERING RHODIUM FROM HYDROFORMYLATION PRODUCTS
PROCEDE POUR RECUPERER DU RHODIUM A PARTIR DE PRODUITS D'HYDROFORMYLATION

(30) Priorität: 24.03.2000 DE 10014844
(43) Veröffentlichungstag der Anmeldung: 15.01.2003
(73) Patentinhaber: Celanese Chemicals Europe GmbH, 46147 Oberhausen (DE)
(72) Erfinder: WIEBUS, Ernst, 46147 Oberhausen (DE); SCHALAPSKI, Kurt, 46147 Oberhausen (DE); MERTL, Michael, 46147 Oberhausen (DE); LUKAS, Rainer, 45133 Essen (DE); FISCHER, Richard, Louisville, KY 40241 (US)
(86) Internationale Anmeldenummer: PCT/EP2001/002848
(87) Internationale Veröffentlichungsnummer: WO 2001/072679

(56) Entgegenhaltungen:
- DE-A- 1 954 315
- DE-A- 2 262 852
- DE-A- 2 311 388
- US-A- 4 388 279
- PATENT ABSTRACTS OF JAPAN vol. 016, no. 049 (C-0908), 7. Februar 1992 (1992-02-07) & JP 03 253522 A (MITSUBISHI KASEI CORP), 12. November 1991 (1991-11-12)
- DATABASE WPI Section Ch, Week 198838 Derwent Publications Ltd., London, GB; Class E19, AN 1988-268742 XP002169277 & JP 63 197543 A (KANSAI NETUKAGAKU K), 16. August 1988 (1988-08-16)
- DATABASE WPI Section Ch, Week 198833 Derwent Publications Ltd., London, GB; Class E14, AN 1988-230339 XP002169278 & JP 63 162044 A (DAICEL CHEM IND LTD), 5. Juli 1988 (1988-07-05)

## Beschreibung

Die vorliegende Erfindung betrifft ein verbessertes Verfahren zur Wiedergewinnung von Rhodium aus den Produkten der Hydroformylierung (Oxosynthese).

Die Herstellung von Aldehyden und Alkoholen durch katalytische Anlagerung von Kohlenmonoxid und Wasserstoff an olefinische Doppelbindungen ist bekannt. Moderne Verfahren arbeiten mit metallischem Rhodium oder mit Rhodiumverbindungen als Katalysatoren, die allein oder mit komplexbildenden Liganden, z.B. organischen Phosphinen oder Estern der phosphorigen Säure, eingesetzt werden. Unter den Reaktionsbedingungen als Katalysator wirksam sind nach übereinstimmender Auffassung der Fachwelt Hydridocarbonylverbindungen des Rhodiums, die sich durch die allgemeine Formel H[Rh(CO)₄₋ₓLₓ] wiedergeben lassen, wobei L einen Liganden bezeichnet und x gleich 0 oder eine ganze Zahl von 1 bis 3 ist.

Die Verwendung von Rhodiumkatalysatoren hat gegenüber der klassischen Oxosynthese mit Kobaltkatalysatoren eine Reihe Vorteile. Die Aktivität von Rhodiumkatalysatoren ist höher als die von Kobaltkatalysatoren und endständige Olefine werden in Gegenwart von Rhodium (in Form von Rhodiumkomplexverbindungen) in höherem Maße zu unverzweigten Aldehyden umgesetzt, als in Gegenwart von Kobalt. Überdies lassen sich Produktionsanlagen bei Verwendung von Rhodiumkatalysatoren weitgehend problemlos betreiben, das betrifft insbesondere die Durchführung der Synthese und die Ausbringung der Produkte.

Ein für die Wirtschaftlichkeit des Rhodiumverfahrens bestimmender Faktor ist die möglichst verlustfreie Abtrennung und Wiedergewinnung des Edelmetalls, unabhängig davon, ob es mit oder ohne zusätzlichem Komplexbildner als Katalysator eingesetzt wurde. Nach Beendigung der Umsetzung findet sich das Rhodium als Carbonylverbindung, die gegebenenfalls noch weitere Liganden enthält, gelöst im Hydroformylierungsprodukt.

Zur Aufarbeitung wird das Rohprodukt der Synthese unter Freisetzen gelösten Synthesegases zunächst ein- oder mehrstufig auf Normaldruck entspannt. Die Abtrennung des Rhodiums erfolgt entweder unmittelbar aus dem entspannten Rohprodukt oder aus dem Rückstand, der nach Abdestillieren der Aldehyde aus dem Rohprodukt anfällt. Die zweite Variante wird angewandt, wenn der Rhodiumkatalysator außer Kohlenmonoxid noch weitere Liganden, z.B. Phosphine oder Phosphite in komplexer Bindung enthält. Sie kann auch dann genutzt werden, wenn die Hydroformylierung mit Rhodium allein durchgeführt wurde und im Verlauf der Weiterbehandlung des Reaktionsproduktes durch Destillation dafür Sorge getragen wird, dass das Rhodium nicht in Form flüchtiger Verbindungen aus dem Destillationsgut oder dem Destillationsrückstand entweicht.

Unabhängig von der gewählten Form der Aufbereitung des Reaktionsgemisches ist zu berücksichtigen, dass das Edelmetall im Rohprodukt ebenso wie im Destillationsrückstand in einer Konzentration von nur wenigen ppm vorliegt, seine Abtrennung daher sehr umsichtiges Arbeiten erfordert.

Unter diesen Umständen überrascht es nicht, dass die Rückgewinnung von Rhodium aus den Produkten der Oxosynthese einschließlich deren Destillationsrückständen, vielfach untersucht wurde. Die Arbeiten führten zur Entwicklung zahlreicher Verfahren, von denen einige auch Anwendung im technischen Maßstab gefunden haben.

Nach einem in der US-Patentschrift 42 92 196 beschriebenen Verfahren extrahiert man Metalle der 8. Gruppe des Periodensystems, die als Katalysatoren in Form von Metallcarbonylen oder metallorganischen Verbindungen homogen gelöst in Hydroformylierungsprodukten vorliegen, mit wasserlöslichen, Stickstoff enthaltenden Verbindungen. Die Abtrennung erfolgt bei Temperaturen, die zwischen Raumtemperatur und 100°C liegen und in einem Bereich von normalem bis zu 7 MPa erhöhtem Druck. Als stickstoffhaltige Verbindungen werden zur Extraktion Ammoniak, Ammoniumhydroxid und Amine eingesetzt.

Ein weiteres Verfahren zur Abtrennung von Rhodium aus den Produkten der Oxosynthese, das auf der Extraktion mit einem komplexbildenden Reagenz beruht, ist in der EP 0 147 824 B1 beschrieben. Als Extraktionsmittel werden wasserlösliche Sulfonate, oder Carboxylate organischer Phosphine in Form einer wässrigen Lösung, die mit dem Oxorohprodukt nicht mischbar ist, eingesetzt.

In beiden Verfahren werden die in den Hydroformylierungsprodukten vorliegenden Metallcarbonyl- oder metallorganischen Verbindungen ohne vorherige Aufspaltung der koordinativen oder der Metall-Kohlenstoff-Bindungen mit dem Extraktionsmittel behandelt.

Die deutsche Offenlegungsschrift 1 954 315 offenbart ein Verfahren zur Abtrennung von Rhodiumcarbonylkatalysatoren aus Oxoreaktionsgemischen, indem man das rohe Oxoreaktionsgemisch bei Temperaturen von 0 bis 120°C in Gegenwart von Kohlenmonoxid und Wasserstoff unter einem Druck von 5 bis 700 Atmosphären mit einem basischen Ionenaustauscher behandelt.

Die Zusammenfassung der JP 3 253 522 betrifft die Rückgewinnung von Rhodium aus Hydroformylierungsprodukten durch Zugabe von Zink aktivierter Aktivkohle und Wasser und nachträglichem Erhitzen, wobei sich der Rhodiumcarbonylkomplex zersetzt und Rhodium sich auf der Aktivkohle niederschlägt.

US 4,388,279 behandelt die Rhodiumrückgewinnung aus Alkoholen, die das Zielprodukt der Hydroformylierungsreaktion darstellen. Das organische Produkt wird zunächst mit einer wässrigen Ammoniaklösung extrahiert, wobei die Hauptmenge des Rhodiums abgetrennt wird. In dem Alkohol verbliebene Rhodiumspuren werden anschließend auf einem festen Adsorbens, beispielsweise auf Calciumsulfat, niedergeschlagen.

Die deutsche Offenlegungsschrift 2 262.852 befasst sich mit der Rhodiumrückgewinnung aus Carbonylierungsprodukten, beispielsweise aus Essigsäure oder Propionsäure, die durch Umsetzung olefinisch oder acetylenisch ungesättigter Verbindungen mit Kohlenmonoxid und Wasser anfallen. Aus diesen Produkten wird Rhodium durch Behandlung mit Wasserstoff auf einem Träger niedergeschlagen, wobei das Tägermaterial mit Edelmetallen dotiert sein kann.

Nach der Zusammenfassung der JP 63 197 543 verwendet man zur Abtrennung von Rhodium aus organischen Lösungen eine mittels Oxidation aktivierte Aktivkohle, die zusätzlich noch mit einer Organophosphorverbindung, beispielsweise mit Triphenylphosphin, beladen wird.

Die Zusammenfassung der JP 63 162 044 lehrt die Adsorption von Rhodiumspuren aus organischen Lösungen mittels Aktivkohle oder Kieselgel, nachdem bereits die Hauptmenge des Rhodiums vorher abgetrennt worden war.

Die deutsche Offenlegungsschrift 2 311 388 offenbart ein Verfahren zur Rhodiumrückgewinnung aus hochsiedenden Destillationsrückständen durch Behandlung dieser Destillationsrückstände mit einem selektiven Adsorptionsmittel. Nach Abtrennung des hochsiedenden Destillationsrückstands wird der Rhodiumkomplex mit einem polaren organischen Lösungsmittel aus dem Adsorbens herausgewaschen.

Die bekannten Verfahren erlauben es, bei technischer Durchführung bis zu 90% des ursprünglich eingesetzten Rhodiums wiederzugewinnen, der Rest des Edelmetalls geht verloren. Probleme bei der Aufarbeitung der Rhodiumextrakte treten bisweilen dadurch auf, dass die Rhodiumkonzentration in den wässrigen Lösungen gering ist und entweder große Flüssigkeitsvolumina behandelt oder die Lösungen zuvor konzentriert werden müssen. Weitere Schwierigkeiten können sich bei der Wiederverwendung des als Metall oder als Verbindung zurückgewonnenen Rhodiums ergeben. Daher besteht Interesse daran, die Abtrennung auch geringer Rhodiumreste aus den Produkten der Oxosynthese zu vervollkommnen, die Rhodiumverluste weiter zu verringern und das Metall in einer Form zu gewinnen, die seine problemlose Rückumwandlung in den Katalysator erlaubt.

Die Erfindung löst die vorstehend beschriebene Aufgabe durch ein Verfahren zur Rückgewinnung von Rhodium, das in den Aldehyden oder Aldehydgruppen enthaltenden mehrfunktionellen Verbindungen der lediglich entspannten Rohprodukte der Hydroformylierung olefinisch ungesättigter Verbindungen enthalten ist, wobei die Hydroformylierungsprodukte in Gegenwart von Aktivkohle, oberflächenreicher Polykieselsäure, oberflächenreichem Aluminiumoxid oder oberflächenreichem Aluminiumoxidhydrat als festes Adsorbens und in Gegenwart von Wasserstoff bei Drücken von 0,1 bis 15 MPa auf Temperaturen von 50 bis 200°C erhitzt werden.

Überraschenderweise gelingt es nach dem neuen Verfahren, auch in sehr geringer Konzentration als Verbindung gelöst vorliegendes Rhodium aus dem Reaktionsgemisch zu isolieren. Die beanspruchte Arbeitsweise stellt sicher, dass ein sehr hoher Anteil des Rhodiums abgetrennt wird. Von besonderer Bedeutung ist in diesem Zusammenhang, dass die thermische Behandlung des Einsatzmaterials schonend, bei mäßigen Temperaturen erfolgt. Unerwünschte Nebenreaktionen sind daher nicht zu befürchten.

Einsatzmaterial sind die Produkte der Hydroformylierung olefinisch ungesättigter Verbindungen. Unter dieser Bezeichnung werden Aldehyde, bzw. Aldehydgruppen enthaltende mehrfunktionelle Verbindungen verstanden. Im Vordergrund des neuen Verfahrens steht die Wiedergewinnung von Rhodium aus der Hydroformylierung von Olefinen mit 2 bis 12 Kohlenstoffatomen, entsprechend der wirtschaftlichen Bedeutung der aus ihnen hergestellten Aldehyde. Neben Aldehyden, den gesättigten und ungesättigten Kondensationsprodukten und Alkoholen können die zu verarbeitenden Gemische auch noch Lösungsmittel enthalten.

Rhodium liegt in den Hydroformylierungsprodukten als Carbonyl- oder Hydridocarbonylverbindung vor. Das erfindungsgemäße Verfahren zeichnet sich dadurch aus, dass es die Behandlung von Reaktionsgemischen erlaubt, die Rhodium in sehr weiten Konzentrationsbereichen enthalten. Es wird mit Erfolg sowohl bei Konzentrationen von 0,5 Gew.-ppm Rhodium als auch bei Konzentrationen von 500 Gew.-ppm Rhodium, bezogen auf das Einsatzmaterial, angewandt.

Die Anwendung des neuen Verfahrens ist nicht auf Mischungen beschränkt, die Rhodium lediglich als Carbonyle enthalten. Es können vielmehr auch Produkte der Oxosynthese eingesetzt werden, zu deren Herstellung Komplexverbindungen des Rhodiums eingesetzt wurden, die neben Kohlenmonoxid noch weitere Liganden enthalten. Zu diesen Liganden zählen insbesondere Verbindungen des dreiwertigen Phosphors wie Phosphine und Phosphite, die über freie Elektronenpaare verfügen und daher zur Ausbildung von Koordinationsverbindungen mit dem Edelmetall befähigt sind. Vor der Behandlung derartiger Produkte nach dem erfindungsgemäßen Verfahren ist es jedoch erforderlich, diese Liganden in Verbindungen zu überführen, die mit Rhodium keine Komplexbindung eingehen. Nach einer bewährten Arbeitsweise wandelt man zu diesem Zweck die Phosphor(III)-verbindungen durch Oxidation in Phosphor(V)-verbindungen um.

Erfindungsgemäß werden die Produkte der Oxosynthese in Gegenwart eines der obengenannten Adsorbentien thermisch behandelt, d.h. auf 50 bis 200°C erhitzt, um das in gebundener Form, in untergeordnetem Maße gegebenenfalls aber auch als feine Metallpartikel vorliegende Rhodium aus dem Reaktionsgemisch abzuscheiden. Niedrigere Temperaturen als 50°C sind zwar nicht ausgeschlossen, sie erweisen sich aber wegen der geringen Reaktions-(Abscheidungs-)geschwindigkeit u.a. aus wirtschaftlichen Gründen, häufig als ungeeignet. Höhere Temperaturen ergeben nur selten eine Verbesserung der Abscheidungsrate. Sie können jedoch durch das Auftreten von Folgereaktionen, die z.B. zur Bildung unerwünschter Kondensationsprodukte führen, die Ausbeute der Zielverbindungen, der Aldehyde, deutlich mindern. Besonders bewährt hat es sich, die thermische Behandlung der Hydroformylierungsprodukte bei 70 bis 150°C durchzuführen.

Unter dem Begriff Adsorbens werden feste Stoffe verstanden, die aufgrund ihrer großen Oberfläche in der Lage sind, in gelöster Form vorliegende Rhodiumverbindungen und, wie bereits gesagt, auch feine Metallteilchen aufzunehmen und anzureichern. Die Anreicherung erfolgt weitgehend selektiv, d.h. durch die Oberflächenkräfte des Adsorbens werden bevorzugt Rhodiumverbindungen gebunden, während die in großem Überschuss vorliegenden organischen Begleitstoffe, wenn überhaupt, dann nur in untergeordnetem Maße durch das Adsorbens fixiert werden.

Als Adsorbentien werden die in der chemischen Praxis, sowohl im Labor als auch in technischen Anlagen gebräuchlichen Materialien eingesetzt nämlich Aktivkohle, oberflächenreiche Polykieselsäuren wie Silicagele (Kiesel-Xerogele), und oberflächenreiche Aluminiumoxide und Aluminiumoxidhydrate. Welches der genannten Adsorbentien im Einzelfall eingesetzt wird hängt davon ab, in welcher Form das Rhodium wiedergewonnen werden soll. Steht die Wiedergewinnung als Verbindung im Vordergrund, dann werden Adsorbentien auf Silicium- und Aluminiumbasis im allgemeinen bevorzugt, weil sie auch der Behandlung mit aggressiven Chemikalien zur Abtrennung des Rhodiums widerstehen und daher wiederholt eingesetzt werden können.

Die Adsorbentien können als einheitliche Stoffe oder als Gemische unterschiedlicher Substanzen im neuen Prozess eingesetzt werden. Auf diese Weise ist es möglich, das erfindungsgemäße Verfahren individuellen Anforderungen anzupassen und auch spezielle Aufgabenstellungen zu lösen. Im allgemeinen wird man Mischungen solcher Adsorbentien verwenden, die sich lediglich im physikalischen Verhalten, z.B. in den Oberflächeneigenschaften, unterscheiden, deren chemischer Aufbau jedoch gleich oder ähnlich ist. Dadurch stellt man u.a. sicher, dass sich die mit dem Edelmetall beladenen Substanzgemische unproblematisch zu Adsorbens und Metall aufbereiten lassen.

Besonders einfach gestaltet sich die Rückgewinnung des Rhodiums in metallischer oder oxidischer Form unter Verwendung von Aktivkohle. Hierbei wird die mit Rhodium beladene Aktivkohle verbrannt und das Edelmetall bleibt, fein verteilt, in elementarer Form oder als Oxid zurück. Es kann unmittelbar in einem gesonderten Reaktionsschritt oder im Reaktionsgemisch selbst, in den Katalysator überführt werden.

Nach der erfindungsgemäßen Arbeitsweise führt man die thermische Behandlung der Rhodium enthaltenden Hydroformylierungsprodukte nicht nur in Gegenwart des Adsorbens, sondern bei gleichzeitiger Anwesenheit von Wasserstoff durch. Der Wasserstoff wird, bezogen auf Rhodium, im Überschuss, in reiner Form oder im Gemisch mit inerten Gasen eingesetzt; die Verwendung von reinem Wasserstoff wird im allgemeinen bevorzugt. Die Wasserstoffmenge kann in weiten Grenzen variiert werden.

Es empfiehlt sich, je mol Rhodium 100 bis 2000, insbesondere 300 bis 1200 mol Wasserstoff anzuwenden. Besonders bemerkenswert ist, dass die im Reaktionsgemisch enthaltenen Aldehyde durch die thermische Behandlung der Hydroformylierungsprodukte in Anwesenheit von Wasserstoff nicht hydriert werden. Diese Reaktion ist nämlich wegen der Bildung katalytisch wirksamen metallischen Rhodiums nicht auszuschließen.

Das neue Verfahren kann sowohl drucklos als auch bei Drücken von 0.1 bis 15 MPa durchgeführt werden. Besonders bewährt haben sich Drücke im Bereich von 5 bis 10 MPa.

Zur praktischen Durchführung der Rhodiumabtrennung nach dem erfindungsgemäßen Verfahren bringt man das rhodiumhaltige Gemisch in einem geeigneten Reaktor mit dem Adsorbens und mit Wasserstoff in Kontakt. Das Adsorbens kann z.B. in einem Rohrreaktor als Festbett angeordnet werden, durch das die Rhodium enthaltende Phase strömt. Das Festbettvolumen und die Größe der Adsorbenspartikel können in weiten Bereichen variiert und so den gewählten Reaktionsbedingungen und den Verfahrensgegebenheiten, wie der gewünschten Strömungsgeschwindigkeit, angepasst werden. Es hat sich bewährt, Raumgeschwindigkeit im Bereich von 0,1 bis 2,5, insbesondere von 1,0 bis 1,5 (V_{Reak.-gemisch}/[V_{Adsorbens} · h]) einzuhalten.

Nach einer anderen Ausführungsform der erfindungsgemäßen Arbeitsweise suspendiert man das Adsorbens, das in diesem Fall sehr feinteilig sein kann, in dem rhodiumhaltigen Medium. Es ist zweckmäßig, die Suspension ständig zu bewegen, z.B. durch Rühren oder Einleiten eines Gases wie Wasserstoff, um einen innigen Kontakt zwischen der flüssigen Phase und dem Adsorbens zu erzielen. Das Massenverhältnis von flüssiger Phase zu Adsorbens kann weitgehend frei und somit den individuellen Erfordernissen entsprechend eingestellt werden. Es hat sich bewährt, je 100 Gew.-teile rhodiumhaltiges Gemisch 1 bis 5, vorzugsweise 1,5 bis 3,5 Gew.-teile Adsorbens einzusetzen. Für die Realisierung dieser Verfahrensvariante eignen sich z.B. Rührkessel oder Autoklaven.

Die Reaktionszeit ist abhängig von der Rhodiumkonzentration im Einsatzmaterial. Sie wird weiterhin durch die eingesetzte Wasserstoffmenge sowie durch Reaktionstemperatur und -druck bestimmt. Unter gleichen Reaktionsbedingungen erfordern höhere Rhodiumkonzentrationen längere Behandlungszeiten als niedrige Konzentrationen. Hohes Wasserstoffangebot, hohe Temperaturen und Drücke fördern die Rhodiumabscheidung auf das Adsorbens. In den meisten Fällen genügt eine einmalige thermische Behandlung des Einsatzgemisches. Das gilt für die Reaktionsdurchführung in Gegenwart von Wasserstoff. Selbstverständlich kann das Einsatzgemisch rezirkuliert werden, um durch mehrfache Behandlung der organischen Phase die Rhodiumabtrennung zu vervollständigen. Ebenso ist es möglich, die Adsorption in mehreren Stufen durchzuführen. Sowohl absatzweise als auch kontinuierliche Reaktionsführung ist möglich.

Das neue Verfahren hat sich ausgezeichnet zur Abscheidung von Rhodium, das als Verbindung gelöst in Produkten der Hydroformylierung vorliegt, bewährt. Abgesehen von der Einfachheit seiner technischen Realisierung ist besonders bemerkenswert, dass es den Einsatz von Gemischen erlaubt, die Rhodium in sehr weiten Konzentrationsbereichen enthalten. Es wird mit Erfolg sowohl bei Konzentrationen von 0,5 Gew.-ppm als auch von 500 Gew.-ppm Rhodium im Einsatzmaterial angewandt. Besonders bewährt hat es sich zur Behandlung von Hydroformylierungsgemischen, in denen Rhodium in Konzentrationen von 2,5 bis 250 Gew.-ppm vorliegt. Je nach Art des Einsatzmaterials und den gewählten Reaktionsbedingungen gelingt es bei Anwendung der beanspruchten Arbeitsweise, bis zu etwa 98% des Rhodiums auf dem Adsorbens abzuscheiden und die Rhodiumkonzentration im behandelten Substrat bis unter 0,1 Gew.-ppm zu senken.

In den folgenden Beispielen wird das erfindungsgemäße Verfahren näher beschrieben. Es ist selbstverständlich nicht auf die wiedergegebenen Ausführungsformen beschränkt.

### Beispiele

### Beispiel 1

Die Abscheidung des Rhodiums auf das Adsorbens erfolgte in einem Rohrreaktor bei 60°C und 7 MPa Druck in Gegenwart von Wasserstoff. Zum Einsatz gelangte ein nicht vorbehandeltes (d.h. lediglich entspanntes) Reaktionsgemisch mit einem Rhodium-Gehalt von 4,2 Gew.-ppm, das bei der Hydroformylierung von Buten unter Verwendung von Rhodium als Katalysator erhalten worden war. Das Ausgangsgemisch wurde am Kopf des Reaktors aufgegeben. Es strömte mit einer Raumgeschwindigkeit von 1,5 V/[V·h] durch gekörnte Aktivkohle der Marke NORIT, Typ GAC 830 plus als Adsorbens, die am Reaktorfuß auf einer porösen Platte angeordnet war. Das behandelte Produkt enthielt nach einmaligem Durchgang durch die Aktivkohle noch 0,08 Gew.-ppm Rhodium, entsprechend einer Abscheidungsrate von 98%.

### Beispiel 2

Dieser Versuch wurde in der Apparatur und unter den Reaktionsbedingungen (Temperatur, Druck, Gegenwart von Wasserstoff, Raumgeschwindigkeit) des Beispiels 1 durchgeführt. Zum Einsatz gelangte ein nicht vorbehandeltes Reaktionsgemisch mit einem Rhodium-Gehalt von 2,82 Gew.-ppm, das bei der Hydroformylierung von Octen unter Verwendung von Rhodium als Katalysator erhalten worden war. Als Adsorbens diente wiederum Aktivkohle der Marke NORIT, Typ GAC 830 plus. Das behandelte Produkt enthielt nach einmaligem Durchgang durch die Aktivkohle noch 0,099 Gew.-ppm Rhodium, entsprechend einer Abscheidungsrate von 96,5%.

### Beispiel 3

Dieser Versuch wurde in der Apparatur des Beispiels 1 bei 80°C, drucklos in Abwesenheit von Wasserstoff durchgeführt. Zum Einsatz gelangte ein nicht vorbehandeltes Reaktionsgemisch mit einem Rhodium-Gehalt von 1,94 Gew.-ppm, das bei der Hydroformylierung von Octen erhalten worden war. Raumgeschwindigkeit und Adsorbens entsprachen denen der Beispiele 1 und 2. Nach einmaligem Durchgang durch die Aktivkohle enthielt das behandelte Produkt noch 0,21 Gew.-ppm Rhodium, entsprechend einer Abscheidungsrate von 89,2%.

### Beispiel 4

Die Abscheidung des Rhodiums auf das Adsorbens erfolgte in einem Rührautoklaven bei 80°C und 7 MPa Druck in Gegenwart von Wasserstoff. Zum Einsatz gelangten 500 g eines nicht vorbehandelten Reaktionsgemisches, das bei der Hydroformylierung von Octen unter Verwendung von Rhodium als Katalysator erhalten worden war und 2,21 Gew.-ppm Rhodium enthielt. In diesem Gemisch wurden 12,5 g Aktivkohle (d.h. 2,5%, bezogen auf das Gemisch) der Marke NORIT, Typ GAC 830 plus in Pulverform suspendiert. Nach 2 h Behandlungsdauer wurde ein Produkt erhalten, das noch 0,27 Gew.-ppm Rhodium aufwies, entsprechend einer Abscheidungsrate von 88%.

## Patentansprüche

1. Verfahren zur Rückgewinnung von Rhodium, das in den Aldehyden oder Aldehydgruppen enthaltenden mehrfunktionellen Verbindungen der lediglich entspannten Rohprodukte der Hydroformylierung olefinisch ungesättigter Verbindungen enthalten ist, wobei die Hydroformylierungsprodukte in Gegenwart von Aktivkohle, oberflächenreicher Polykieselsäure, oberflächenreichem Aluminiumoxid oder oberflächenreichem Aluminiumoxidhydrat als festes Adsorbens und in Gegenwart von Wasserstoff bei Drücken von 0,1 bis 15 MPa auf Temperaturen von 50 bis 200°C erhitzt werden.

2. Verfahren nach Anspruch 1, **dadurch gekennzeichnet, dass** die Hydroformylierungsprodukte auf Temperaturen von 70 bis 150°C erhitzt werden.

3. Verfahren nach Anspruch 1, **dadurch gekennzeichnet, dass** der Wasserstoff in reiner Form oder in Mischung mit inerten Gasen eingesetzt wird.

4. Verfahren nach einem oder mehreren der Ansprüche 1 bis 3, **dadurch gekennzeichnet, dass** Wasserstoff, bezogen auf Rhodium, mindestens in stöchiometrischer Menge eingesetzt wird.

5. Verfahren nach einem oder mehreren der Ansprüche 1 bis 4, **dadurch gekennzeichnet, dass** je mol Rhodium 100 bis 2000 mol Wasserstoff angewandt werden.

6. Verfahren nach Anspruch 5, **dadurch gekennzeichnet, dass** je mol Rhodium 300 bis 1200 mol Wasserstoff angewandt werden.

7. Verfahren nach einem oder mehreren der Ansprüche 1 bis 6, **dadurch gekennzeichnet, dass** die thermische Behandlung der Hydroformylierungsprodukte bei Drücken von 5 bis 10 MPa durchgeführt wird.

8. Verfahren nach einem oder mehreren der Ansprüche 1 bis 7, **dadurch gekennzeichnet, dass** die thermische Behandlung der Hydroformylierungsprodukte an einem als Festbett angeordneten Adsorbens erfolgt.

9. Verfahren nach Anspruch 8, **dadurch gekennzeichnet, dass** bei der thermischen Behandlung der Hydroformylierungsprodukte Raumgeschwindigkeiten von 0,1 bis 2,5 V_{Reak.-gemisch}/[V_{Adsorbens} · h] eingehalten werden.

10. Verfahren nach Anspruch 9, **dadurch gekennzeichnet, dass** bei der thermischen Behandlung der Hydroformylierungsprodukte Raumgeschwindigkeiten von 1,0 bis 1,5 V_{Reak-gemisch}/[V_{Adsorbens} · h] eingehalten werden.

11. Verfahren nach einem oder mehreren der Ansprüche 1 bis 7, **dadurch gekennzeichnet, dass** das Adsorbens im Hydroformylierungsprodukt suspendiert ist.

12. Verfahren nach Anspruch 11, **dadurch gekennzeichnet, dass** je 100 Gew.-teile Rhodium enthaltendes Hydroformylierungsprodukt 1 bis 5 Gew.-teile Adsorbens suspendiert werden.

13. Verfahren nach Anspruch 12, **dadurch gekennzeichnet, dass** je 100 Gew.-teile Rhodium enthaltendes Hydroformylierungsprodukt 1,5 bis 3,5 Gew.-teile Adsorbens suspendiert werden.

## Claims

1. A process for recovering rhodium present in the aldehyde or aldehyde group-containing polyfunctional compounds of the merely decompressed crude products of the hydroformylation of olefinically unsaturated compounds, which comprises heating the hydroformylation products in the presence of activated carbon, high surface-area polysilicic acid, high surface-area aluminum oxide or high surface-area aluminium oxide hydrate as solid adsorbent and in the presence of hydrogen under pressures of from 0.1 to 15 MPa to temperatures of from 50 to 200°C.

2. The process as claimed in claim 1, wherein the hydroformylation products are heated to temperatures of from 70 to 150°C.

3. The process as claimed in claim 1, wherein the hydrogen is employed in pure form or mixed with inert gases.

4. The process as claimed in one or more of claims 1 to 3, wherein hydrogen is employed in at least stoichiometric amount relative to rhodium.

5. The process as claimed in one or more of claims 1 to 4, wherein from 100 to 2000 mol of hydrogen are used per mol of rhodium.

6. The process as claimed in claim 5, wherein from 300 to 1200 mol of hydrogen are used per mol of rhodium.

7. The process as claimed in one or more of claims 1 to 6, wherein the thermal treatment of the hydroformylation products is carried out under pressures of from 5 to 10 MPa.

8. The process as claimed in one or more of claims 1 to 7, wherein the thermal treatment of the hydroformylation products takes place on an adsorbent disposed as fixed bed.

9. The process as claimed in claim 8, wherein space velocities of from 0.1 to 2.5 V_{react.mixt}./[V_{adsorbent} · h] are maintained in the thermal treatment of the hydroformylation products.

10. The process as claimed in claim 9, wherein space velocities of from 1.0 to 1.5 V_{react.mixt}/[V_{adsorbent} · h] are maintained in the thermal treatment of the hydroformylation products.

11. The process as claimed in one or more of claims 1 to 7, wherein the adsorbent is suspended in the hydroformylation product.

12. The process as claimed in claim 11, wherein from 1 to 5 parts by weight of adsorbent are suspended per 100 parts by weight of rhodium-containing hydroformylation product.

13. The process as claimed in claim 12, wherein from 1.5 to 3.5 parts by weight of adsorbent are suspended per 100 parts by weight of rhodium-containing hydroformylation product.

## Revendications

1. Procédé de récupération du rhodium contenu dans les composés polyfonctionnels qui contiennent des aldéhydes ou des groupes aldéhyde dans des produits bruts, uniquement détendus, d'hydroformylation de composés oléfiniquement insaturés, les produits d'hydroformylation étant chauffés à des températures de 50 à 200°C en présence de charbon actif, d'acide polysilicique à haute surface spécifique, d'oxyde d'aluminium à haute surface spécifique ou d'oxyde d'aluminium hydraté à haute surface spécifique qui servent d'adsorbant solide et en présence d'hydrogène à des pressions de 0,1 à 15 MPa.

2. Procédé selon la revendication 1, **caractérisé en ce que** les produits d'hydroformylation sont chauffés à des températures de 70 à 150°C.

3. Procédé selon la revendication 1, **caractérisé en ce que** l'hydrogène est utilisé sous forme pure ou en mélange avec des gaz inertes.

4. Procédé selon l'une ou plusieurs des revendications 1 à 3, **caractérisé en ce que** l'hydrogène peut être utilisé en quantité au moins stoechiométrique par rapport au rhodium.

5. Procédé selon l'une ou plusieurs des revendications 1 à 4, **caractérisé en ce que** par mole de rhodium, on utilise de 100 à 2 000 moles d'hydrogène.

6. Procédé selon la revendication 5, **caractérisé en ce que** l'on utilise de 300 à 1 200 moles d'hydrogène par mole de rhodium.

7. Procédé selon l'une ou plusieurs des revendications 1 à 6, **caractérisé en ce que** le traitement thermique des produits d'hydroformylation est réalisé à des pressions de 5 à 10 MPa.

8. Procédé selon l'une ou plusieurs des revendications 1 à 7, **caractérisé en ce que** le traitement thermique des produits d'hydroformylation s'effectue sur un adsorbant disposé sous la forme d'un lit fixe.

9. Procédé selon la revendication 8, **caractérisé en ce que** lors du traitement thermique des produits d'hydroformylation, on maintient des vitesses spatiales de 0,1 à 2, 5 V_{mélange réact}. /[V_{adsorbant} . h].

10. Procédé selon la revendication 9, **caractérisé en ce que** lors du traitement thermique des produits d'hydroformylation, on maintient des vitesses spatiales de 1,0 à 1,5 V_{mélange réact}./[V_{adsorbant} . h].

11. Procédé selon l'une ou plusieurs des revendications 1 à 7, **caractérisé en ce que** l'adsorbant est mis en suspension dans le produit d'hydroformylation.

12. Procédé selon la revendication 11, **caractérisé en ce que** l'on met en suspension de 1 à 5 parties en poids d'adsorbant pour 100 parties en poids de produit d'hydroformylation qui contient du rhodium.

13. Procédé selon la revendication 12, **caractérisé en ce que** l'on met en suspension de 1,5 à 3,5 parties en poids d'adsorbant pour 100 parties en poids de produit d'hydroformylation qui contient du rhodium.
